# FASCICULE DE BREVET EUROPEEN

(11) **EP 0 663 959 B1**
(45) Date de publication et mention de la délivrance du brevet: **05.03.1997**
(21) Numéro de dépôt: 93922002.6
(22) Date de dépôt: 06.10.1993
(51) Int. Cl.: C12Q 1/04, C12Q 1/10, C12N 1/20

(54) **NOUVEAU MILIEU DE CULTURE POUR LA MISE EN EVIDENCE DE $i(E. COLI) ET PROCEDE POUR SON UTILISATION**
NEUES KULTURMILIEU ZUM NACHWEISS E. COLI UND VERFAHREN ZU IHRER VERWENDUNG
NOVEL CULTURE MEDIUM FOR THE ENUMERATION OF $i(E. COLI) AND METHOD OF USE

(30) Priorité: 07.10.1992 FR 9211879
(43) Date de publication de la demande: 26.07.1995
(73) Titulaire: Rambach, Alain, F-75006 Paris (FR)
(72) Inventeur: Rambach, Alain, F-75006 Paris (FR)
(74) Mandataire: Ahner, Francis
(86) Numéro de dépôt international: FR9300988
(87) Numéro de publication internationale: WO9408043

(56) Documents cités:
- LETTERS IN APPLIED MICROBIOLOGY vol. 13, no. 4 , 1 Octobre 1991 , BLACKPOOL UK pages 212 - 215 I.D. OGDEN ET AL. 'An evaluation of fluorogenic and chromogenic assays for the direct enumeration od Escherichia coli.'

## Description

La présente invention concerne un nouveau milieu de culture destiné notamment à la mise en évidence de Escherichia coli.

Il existe actuellement pour la mise en évidence de Escherichia coli qui est l'un des microorganismes dont la détection est la plus fréquente, un certain nombre de milieux de culture.

La détection de E. coli est très importante notamment dans les industries alimentaires, au niveau du contrôle des eaux mais également en médecine, sachant que dans ce milieu E. coli peut être un agent pathogène mais également un agent mettant en évidence certains types de contamination. Compte tenu de la diversité des applications et des conditions dans lesquelles doit se faire la détection, il est donc nécessaire de pouvoir disposer d'un milieu de détection qui soit simple et rapide d'emploi mais également qui soit fiable.

Or, il ressort des essais qui ont été effectués que la plupart des milieux de culture utilisés actuellement, s'ils permettent de détecter un très grand nombre de souches d'Escherichia coli, ne permettent néanmoins pas de détecter d'une manière simple 100 % des contaminations. Or, on sait que généralement on ne peut pas se contenter d'une probabilité de l'ordre de 90 %, car dans la plupart des cas ce type de probabilité est tout à fait insupportable pour des raisons évidentes. Les microorganismes pathogènes qui peuvent être présents dans ces échantillons non contre-sélectionnés pourront avoir tendance à se répandre parce que ne pouvant pas être mis en évidence par les méthodes traditionnelles.

C'est pourquoi la présente invention constitue un progrès significatif dans la mesure où elle propose des milieux de culture permettant de mettre en évidence d'une manière simple un nombre beaucoup plus grand de contaminations de E. coli par rapport aux milieux existants.

Plus particulièrement, il s'agit d'un milieu de culture destiné à la mise en évidence de E. coli caractérisé en ce qu'il comporte outre un milieu de culture pour E. coli à titre d'agent chromogène une combinaison de :
- un composé chromogène dérivé d'acide indolyl-glucuronique ou de ses sels substrats de l'enzyme GUS et
- un dérivé ou sel d'acide alkyle, alcényle ou aryle glucuronique, à l'exception des dérivés chromogènes de l'acide glucuronique.

Les milieux de culture pour la mise en évidence de Escherichia coli sont connus (voir notamment "Letters in Applied Microbiology 13, 212-215, 1991) et ne seront pas redécrits en détail ci-après, mais seront mentionnés uniquement dans les exemples.

En ce qui concerne les composés chromogènes, il s'agit d'agents chromogènes utilisés pour certains d'entre eux déjà dans la détection de E. coli et qui mettent en oeuvre des dérivés d'acide indolyl-glucuronique. Ces composés chromogènes sont des substrats de l'enzyme-bêta-D-glucuronidase ou GUS qui par scission du substrat conduit à un dérivé coloré et/ou fluorescent par exemple.

Parmi ces dérivés de l'acide indolyl-glucuronique, il faut plus particulièrement citer les dérivés des acides halogéno-3-indolyl glucuronique et leurs sels correspondants et plus particulièrement encore les composés choisis parmi l'acide 5-bromo-4-chloro-3-indolyl-bêta-D-glucuronique, les sels de sodium et de cyclohexylammonium, l'acide 5-bromo-6-chloro-3-indolyl-bêta-D-glucuronique les sels de sodium et de cyclohexylammonium, l'acide 6-chloro-3-indolyl-bêta-D-glucuronique, l'acide 5-bromo-3-indolyl bêta-D-glucuronique, l'acide 3-indolyl-bêta-D-glucuronique, leurs sels de sodium et de cyclohexylammonium.

Ces composés sont utilisés selon la présente invention en combinaison avec des dérivés alkylés, alcénylés et/ou arylés non-chromogènes de l'acide glucuronique. Il peut s'agir de dérivés alkylés ou alcénylés en C₁ à C₃ notamment méthylés ou des dérivés arylés de type phényl ou phényl substitué ainsi que les sels correspondants. Parmi les composés les plus efficaces selon la présente invention, il faut citer tout particulièrement l'acide phénylglucuronique de même que ses sels notamment ses sels alcalins, alcalino-terreux et ses sels dérivés d'ammonium tels que par exemple les sels de type cyclohexylammonium.

Suivant la nature de l'agent chromogène utilisé, les colorations qui mettront en évidence la présence de E. coli pourront varier, l'un des intérêts de ce procédé est qu'il permet avec des concentrations en chromogène relativement peu élevées d'obtenir des détections des contaminations de E. coli voisines de 100 %.

D'une manière préférentielle, le rapport pondéral composé chromogène/dérivé d'acide glucuronique est compris entre 1/1 et 1/10, de préférence compris entre 1/1 et 1/3.

Par exemple, on pourra utiliser des concentrations de composés chromogènes inférieures ou égales à 50 mg/l et éventuellement de l'ordre de 30 mg/l, avec des quantités de dérivés d'acide glucuronique voisines de 100 mg/l.

Le milieu selon l'invention pourra en outre être avantageusement supplémenté en phosphate, de préférence à une concentration, supérieure à 1 g/l, plus particulièrement comprise entre 4 g/l et 10 g/l.

La présente invention concerne également un procédé de détection de souches de E. coli dans un prélèvement quel qu'il soit caractérisé en ce qu'on inocule le milieu de culture tel que décrit précédemment avec ledit prélèvement et/ou un inoculum provenant dudit prélèvement et en ce que l'on met au moins en évidence la coloration ou la fluorescence caractéristique de la présence de E. coli.

Ce milieu de culture selon l'invention présente également par rapport aux milieux de la technique antérieure l'avantage de pouvoir être mis en oeuvre selon l'une quelconque des modalités connues dans ce type de technologie. En particulier, il est possible avec les milieux selon l'invention de prévoir une inoculation dans la masse du milieu.

Cet aspect de l'invention est d'autant plus inattendu que les chromogènes dérivés de l'acide indolylglucuronique ne fonctionnant pas en conditions anaérobies, on aurait pu croire que le procédé d'inoculation dans la masse soit incompatible avec leur utilisation dans le milieu selon l'invention.

Enfin, on constate expérimentalement que grâce au milieu de culture selon la présente invention, l'apparition des vrais positifs est accélérée, on élimine des faux négatifs et pratiquement 100 % des contaminations de E. coli sont mises en évidence.

Les exemples ci-après sont destinés à mettre en évidence d'autres caractéristiques et avantages de la présente invention.

### Exemple 1 : Détection de souches de E. coli

Diverses souches de E. coli sont isolées sur le milieu M (en g/l : Extrait de viande 1 ; Extrait de levure 2 ; Peptone 5 ; Chlorure de sodium 5 : Agar 15 ; Desoxycholate de sodium 1,5 ; 5 Bromo-4-Chloro-3-Indolyl glucuronide 0,05).

Les boîtes sont incubées à 37°C et la coloration bleue des colonies est observée après 24 heures d'incubation.

Une des trois souches, E. coli 297-3, n'est mise en évidence qu'après addition de l'acide phénylglucuronique (Tableau 1).

**TABLEAU 1**

| souche E. coli | coloration milieu M | coloration milieu M 100mg/l Acide Phenylglucuronique |
|---|---|---|
| 289-3 | + | + |
| 297-3 | - | + |
| 366-1 | + | + |

L'addition de phosphate permet également d'améliorer la sensibilité du milieu selon l'invention (Tableau 2).

**TABLEAU 2**

| | coloration des colonies de E.coli | |
|---|---|---|
| | incubation 18h | incubation 24h |
| Milieu M | - | +/- |
| | | |
| Milieu M | | |
| Acide phényl glucuronique 100 mg/l | | |
| phosphate 8 g/l | ++ | ++ |

### EXEMPLE 2 : Comparaison avec le milieu PTX

Le milieu PTX comprend comme agent chromogène l'acide 5-bromo-4-chloro-3-indolyl-bêta-D-glucuronique. Il s'agit d'un milieu ayant la même base que le milieu PTG reconnu en France comme utile en matière de milieu de détection de E.coli (Baylac et al. médecine et armées, 1990, 18, p.305-308).

Le milieu PTX (en g/l : Extrait de levure 3 ; Peptone 5 ; phosphate 0,3 ; Tergitol 0,1 ; Agar 15 ; composé chromogène 0,05) a été comparé à un milieu selon l'invention, PTX supplémente en acide phénylglucuronique (0,1 g/l) et en phosphate (8 g/l).

**TABLEAU 3**

| coloration des colonies de E.coli | | |
|---|---|---|
| | incubation 18h | incubation 24h |
| Milieu PTX | - | +/- |
| Milieu PTX | | |
| Acide phényl glucuronique 100 mg/l | | |
| phosphate 8 g/l | ++ | ++ |

### Exemple 3 : Analyse d'échantillons de steaks hachés

Des échantillons de steaks sont examinés par la méthode standard de broyage dilution et étalement.

La gélose suivant l'invention est comparée avec un dispositif commercial sur film où les E. coli sont aussi détectés par simple coloration spécifique.

Les résultats indiquent que la proportion de colonies E. coli détectées suivant l'invention est plus élevée que la technique utilisant un film donnant une coloration spécifique aux colonies de E. coli.

**TABLEAU 4**

| **échantillons de steak haché** | **colonies E. coli détectées sur gélose suivant l'invention** | **colonies E. coli détectées sur film commercial pour E. coli** |
|---|---|---|
| 362 | 0 | 0 |
| 365 | 0 | 0 |
| 371 | 120 | 50 |
| 368 | 102 | 50 |
| 367 | 0 | 56 |
| 363 | 2 | 0 |
| 370 | 40 | 11 |
| 379 | 0 | 0 |
| 380 | 200 | 29 |
| 382 | 3 | 1 |
| 383 | 9 | 4 |
| 384 | 1 | 3 |
| 386 | 3 | 2 |
| 3006 | 0 | 0 |
| 3007 | 0 | 0 |
| 3008 | 192 | 83 |

## Revendications

1. Milieu de culture destiné à la mise en évidence de E. coli caractérisé en ce qu'il comporte outre un milieu de culture pour E. coli à titre d'agents chromogènes une combinaison de :
- un composé chromogène dérivé de l'acide indolylglucuronique substrat de l'enzyme GUS et
- un dérivé d'acide alkyl- alcényl- ou aryl- glucuronique à l'exception des dérivés chromogènes de l'acide glucuronique.

2. Milieu de culture selon la revendication 1, caractérisé en ce que le dérivé d'acide glucuronique est l'acide phénylglucuronique ou ses sels.

3. Milieu de culture selon la revendication 2, caractérisé en ce que le composé chromogène est choisi parmi les acides halogéno-3indolyl glucuronique et les sels correspondants.

4. Milieu de culture selon la revendication 3, caractérisé en ce que le composé chromogène est choisi parmi :
l'acide 5-bromo-4-chloro-3-indolyl-bêta-D-glucuronique, les sels de sodium et de cyclohexylammonium,
l'acide 5-bromo-6-chloro-3-indolyl-bêta-D-glucuronique, les sels de sodium et de cyclohexylammonium,
l'acide 6-chloro-3-indolyl-bêta-D-glucuronique, les sels de sodium et de cyclohexylammonium,
l'acide 5-bromo-3-indolyl-bêta-D-glucuronique, les sels de sodium et de cyclohexylammonium,
l'acide indolyl-bêta-D-glucuronique, les sels de sodium et de cyclohexylammonium.

5. Milieu de culture selon l'une des revendications 1 à 4, caractérisé en ce que le rapport pondéral composé chromogène/dérivé d'acide glucuronique est compris entre 1/1 et 1/10, de préférence compris entre 1/1 et 1/3.

6. Milieu de culture selon la revendication 5, caractérisé en ce qu'il contient au plus 50 mg/l du composé chromogène pour environ 100 mg/l de dérivé d'acide glucuronique.

7. Milieu de culture selon l'une des revendications 1 à 6, caractérisé en ce qu'il comprend en outre du phosphate à une concentration supérieure à 1 g/l, de préférence comprise entre 4 g/l et 10 g/l.

8. Procédé de mise en évidence de E. coli dans un prélèvement, caractérisé en ce qu'on inocule le milieu de culture selon l'une des revendications 1 à 7 avec le prélèvement ou en inoculum provenant dudit prélèvement et en ce qu'elle détecte éventuellement la présence de E. coli.

9. Procédé de mise en évidence de E. coli selon la revendication 8, caractérisé en ce que l'inoculation est effectuée dans la masse du milieu de culture.

## Patentansprüche

1. Kulturmedium zum Nachweis von E. coli, dadurch gekennzeichnet, daß es außer einem Kulturmedium für E. coli als chromogene Agentien enthält eine Kombination aus
- einer chromogenen Verbindung, die von dem Indolylglucuronsäure-Substrat des Enzyms GUS abgeleitet ist, und
- ein Alkyl-, Alkenyl- oder Aryl-Glucuronsäure-Derivat mit Ausnahme der chromogenen Derivate der Glucuronsäure.

2. Kulturmedium nach Anspruch 1, dadurch gekennzeichnet, daß das Glucuronsäure-Derivat die Phenylglucuronsäure oder ihre Salze ist.

3. Kulturmedium nach Anspruch 2, dadurch gekennzeichnet, daß die chromogene Verbindung ausgewählt wird aus den Halogen-3-indolylglucuronsäuren und ihren entsprechenden Salzen.

4. Kulturmedium nach Anspruch 3, dadurch gekennzeichnet, daß die chromogene Verbindung ausgewählt wird aus:
5-Bromo-4-chloro-3-indolyl-β-D-glucuronsäure und ihren Natrium- und Cyclohexylammoniumsalzen,
5-Bromo-6-chloro-3-indolyl-β-D-glucuronsäure und ihren Natrium- und Cyclohexylammoniumsalzen,
6-Chloro-3-indolyl-β-D-glucuronsäure und ihren Natrium- und Cyclohexylammoniumsalzen,
5-Bromo-3-indolyl-β-D-glucuronsäure und ihren Natrium- und Cyclohexylammoniumsalzen und
Indolyl-β-D-glucuronsäure und ihren Natrium- und Cyclohexylammoniumsalzen.

5. Kulturmedium nach einem der Ansprüche 1 bis 4, dadurch gekennzeichnet, daß das Gewichtsverhältnis chromogene Verbindung/Glucuronsäure-Derivat zwischen 1/1 und 1/10, vorzugsweise zwischen 1/1 und 1/3, liegt.

6. Kulturmedium nach Anspruch 5, dadurch gekennzeichnet, daß es höchstens 50 mg/l der chromogenen Verbindung auf etwa 100 mg/l Glucuronsäure-Derivat enthält.

7. Kulturmedium nach einem der Ansprüche 1 bis 6, dadurch gekennzeichnet, daß es außerdem Phosphat in einer Konzentration von mehr als 1 g/l, vorzugsweise zwischen 4 g/l und 10 g/l, enthält.

8. Verfahren zum Nachweis von E. coli in einem Abstrich, dadurch gekennzeichnet, daß man das Kulturmedium nach einem der Ansprüche 1 bis 7 mit dem Abstrich oder einem aus dem Abstrich stammenden Inokulum inokuliert und daß man gegebenenfalls die Anwesenheit von E. coli nachweist.

9. Verfahren zum Nachweis von E.coli nach Anspruch 8, dadurch gekennzeichnet, daß die Inokulation in die Masse des Kulturmediums durchgeführt wird.

## Claims

1. Culture medium intended for the detection of E. coli, characterized in that it contains, in addition to a culture medium for E. coli, as chromogenic agents, a combination of:
- a chromogenic compound derived from indolylglucuronic acid which is a substrate for the GUS enzyme and
- an alkyl-, alkenyl- or arylglucuronic acid derivative, with the exception of the chromogenic derivatives of glucuronic acid.

2. Culture medium according to Claim 1, characterized in that the glucuronic acid derivative is phenylglucuronic acid or its salts.

3. Culture medium according to Claim 2, characterized in that the chromogenic compound is chosen from halo-3-indolylglucuronic acids and the corresponding salts.

4. Culture medium according to Claim 3, characterized in that the chromogenic compound is chosen from:
5-bromo-4-chloro-3-indolyl-beta-D-glucuronic acid, the sodium and cyclohexylammonium salts,
5-bromo-6-chloro-3-indolyl-beta-D-glucuronic acid, the sodium and cyclohexylammonium salts,
6-chloro-3-indolyl-beta-D-glucuronic acid, the sodium and cyclohexylammonium salts,
5-bromo-3-indolyl-beta-D-glucuronic acid, the sodium and cyclohexylammonium salts,
indolyl-beta-D-glucuronic acid, the sodium and cyclohexylammonium salts.

5. Culture medium according to one of Claims 1 to 4, characterized in that the chromogenic compound/glucuronic acid derivative weight ratio is between 1/1 and 1/10, preferably between 1/1 and 1/3.

6. Culture medium according to Claim 5, characterized in that it contains at the most 50 mg/l of the chromogenic compound for about 100 mg/l of glucuronic acid derivative.

7. Culture medium according to one of Claims 1 to 6, characterized in that it comprises, in addition, phosphate at a concentration greater than 1 g/l, preferably between 4 g/l and 10 g/l.

8. Process for the detection of E. coli in a sample, characterized in that the culture medium according to one of Claims 1 to 7 is inoculated with the sample or an inoculum obtained from the said sample and in that it detects, where appropriate, the presence of E. coli.

9. Process for the detection of E. coli according to Claim 8, characterized in that the inoculation is carried out in the bulk of the culture medium.
